Europäisches Patentamt

(19) European Patent Office

Office européen des brevets

(11) Veröffentlichungsnummer: **0 211 299**
**B1**

(12) # EUROPÄISCHE PATENTSCHRIFT

(45) Veröffentlichungstag der Patentschrift:
28.02.90

(21) Anmeldenummer: 86109945.5

(22) Anmeldetag: 19.07.86

(51) Int. Cl. 5: **C 12 N 15/00, C 07 K 13/00,**
**C 12 P 21/02, C 12 N 1/20 //**
**(C12N1/20, C12R1:19)**

(54) Fusionsproteine, Verfahren zu ihrer Herstellung und ihre Verwendung.

(30) Priorität: 27.07.85 DE 3526995

(43) Veröffentlichungstag der Anmeldung:
25.02.87 Patentblatt 87/09

(45) Bekanntmachung des Hinweises auf die Patenterteilung:
28.02.90 Patentblatt 90/09

(84) Bennante Vertragsstaaten:
AT BE CH DE FR GB IT LI LU NL SE

(86) Entgegenhaltungen:
EP-A-0 020 147
EP-A-0 036 776

GENE, Band 9, 1980, Seiten 27-47, Elsevier/North-Holland Biomedical Press; R.A. HALLEWELL et al.: "Plasmid vectors containing the tryptophan operon promoter suitable for efficient regulated expression of foreign genes"

NUCLEIC ACIDS RESEARCH, Band 9, 1981, Seiten 6647-6667, IRL Press Ltd, London, GB; C. YANOFSKY et al.: "The complete nucleotide sequence of the tryptophan operon of Escherichia coli"

NATURE, Band 291, 11. Juni 1981, Seiten 503-506, Macmillan Journals Ltd; J.C. EDMAN et al.: "Synthesis of hepatitis B surface and core antigens in E. coli"

(73) Patentinhaber: HOECHST AKTIENGESELLSCHAFT
Postfach 80 03 20
D-6230 Frankfurt am Main 80 (DE)

(72) Erfinder: Habermann, Paul, Dr.
Heimchenweg 80
D-6230 Frankfurt am Main 80 (DE)
Erfinder: Stengelin, Siegfried, Dr. c/o Massachusetts General Hospital Dept. of Molecular Biology
Fruit Street Boston, MA 02114 (DE)
Erfinder: Wengenmayer, Friedrich, Dr.
Am Seyenbach 38
D-6238 Hofheim am Taunus (DE)

**Beschreibung**

Bei der gentechnischen Herstellung kleinerer eukaryotischer Proteine mit einem Molgewicht bis zu etwa 15 000 Dalton wird in Bakterien häufig nur eine geringe Ausbeute erhalten. Vermutlich werden die gebildeten Proteine durch wirtseigene Proteasen rasch abgebaut. Solche Proteine werden deshalb zweckmäßig als Fusionsproteine, insbesondere mit einem wirtseigenen Proteinanteil, hergestellt, der dann anschliessend abgespalten wird.

Es wurde nun gefunden, daß ein aus nur etwa 70 Aminosäuren bestehender Abschnitt des D-Proteins aus dem trp-Operon von E. coli sich besonders gut zur Bildung von Fusionsproteinen eignet, und zwar im Bereich der Aminosäuresequenz 23 bis 93 (C. Yanofeky et al., Nucleic Acids Res. 9 (1981) 6647), im folgenden auch als "D'-Peptid" bezeichnet. Zwischen dem Carboxy-Terminus dieses Peptids und der Aminosäuresequenz des gewünschten eukaryotischen Proteins befindet sich eine Sequenz aus einer oder mehreren genetisch kodierbaren Aminosäuren, die eine chemische oder enzymatische Abspaltung des gewünschten Proteins erlaubt. In bevorzugten Ausgestaltungen dieser Erfindung schließt sich an den Aminoterminus eine kurze Aminosäuresequenz an, bestehend aus Lys-Ala, gegebenenfalls gefolgt von einer Sequenz von 1 bis 10, insbesondere 1 bis 3 weiteren genetisch kodierbaren Aminosäuren, vorzugsweise von zwei Aminosäuren, insbesondere von Lys-Gly.

Die Erfindung betrifft somit ein Fusionsprotein der allgemeinen Formel

Met - $X_n$ - D' - Y - Z

in der

n   Null oder 1 ist,

X   eine Sequenz von 1 bis 12 genetisch kodierbaren Aminosäuren ist, vorzugsweise Lys-Ala,

D'  eine Sequenz von etwa 70 Aminosäuren im Bereich der Aminosäuresequenz 23 - 93 des D-Peptids im trp-Operon von E. coli ist,

Y   eine Sequenz aus einer oder mehreren genetisch kodierbaren Aminosäuren bedeutet, die eine Abspaltung der folgenden Aminosäuresequenz Z ermöglicht, und

Z   eine Sequenz aus genetisch kodierbaren Aminosäuren ist.

Weitere Aspekte der Erfindung und bevorzugte Ausgestaltungen werden im folgenden wiedergegeben bzw. in den Patentansprüchen definiert.

Es ist natürlich vorteilhaft, wenn der unerwünschte (wirtseigene) Anteil am Fusionsprotein möglichst klein ist, da dann die Zelle nur wenig "Ballast" produziert und dadurch die Ausbeute an dem gewünschten Protein hoch ist. Außerdem entstehen bei der Abspaltung des unerwünschten Anteils weniger Nebenprodukte, was die Aufarbeitung erleichtert. Dem steht entgegen, daß die (vermutete) "Schutzfunktion" des unerwünschten Anteils nur ab einer bestimmten Größe zu erwarten ist. Überraschenderweise zeigte sich nun, daß das erfindungsgemäß gewählte Segment aus dem D-Protein diese Aufgabe erfüllt, obwohl es nur etwa 70 Aminosäuren aufweist.

In vielen Fällen, vor allem bei der bevorzugten Ausführungsform, in der X für Lys-Ala steht oder N-terminal diese Sequenz enthält, wird ein unlösliches Fusionsprotein gebildet. Dieses kann einfach von den löslichen Proteinen abgetrennt werden, was die Aufarbeitung sehr erleichtert und die Ausbeute erhöht. Die Bildung eines unlöslichen Fusionsproteins ist überraschend, da einerseits der bakterielle Anteil mit nur etwa 70 Aminosäuren recht gering ist und andererseits Bestandteil eines in der Wirtszelle in Lösung vorliegenden Proteins ist.

"Etwa 70 Aminosäuren im Bereich der Aminosäuresequenz 23 bis 93 des D-Peptids" besagt, daß in an sich bekannter Weise Variationen möglich sind, also einzelne Aminosäuren weggelassen, ersetzt oder ausgetauscht werden können, ohne daß hierdurch die erfindungsgemäßen Fusionsproteine in ihren Eigenschaften signifikant verändert würden. Solche Variationen sind ebenfalls Gegenstand der Erfindung.

Das gewünschte eukaryotische Protein ist bevorzugt ein biologisch aktives Protein wie ein Hirudin oder eine Vorstufe eines solchen Proteins wie humanes Proinsulin.

Das Fusionsprotein wird durch Expression in einem geeigneten System gewonnen und in der besonders bevorzugten Ausführungsform nach Aufschluß der Wirtszellen aus dem Niederschlag isoliert, in dem es sich aufgrund der Schwerlöslichkeit anreichert. Es ist somit eine leichte Abtrennung von den löslichen Bestandteilen der Zelle möglich.

Als Wirtszellen kommen alle in Betracht, für die Expressionssysteme bekannt sind, also Säugerzellen und Mikroorganismen, vorzugsweise Bakterien, insbesondere E. coli, da ja der bakterielle Anteil des Fusionsproteins ein wirtseigenes Protein von E. coli ist.

Die DNA-Sequenz, die für das erfindungsgemäße Fusionsprotein kodiert, wird in bekannter Weise in einen Vektor eingebaut, der in dem gewählten Expressionssystem eine gute Expression gewährleistet.

In bakteriellen Wirten wählt man zweckmäßig den Promotor und Operator aus der Gruppe Lac, Tac, $P_L$ oder $P_R$ des Phagen λ, hsp, omp oder einen synthetischen Promotor, wie sie beispielsweise in der deutschen Offenlegungsschrift 3 430 683 (Europäische Patentanmeldung 0 173 149) beschrieben sind.

Besonders geeignet ist ein Vektor, der aus dem trp-Operon aus E. coli die folgenden Elemente enthält: den Promotor, den Operator und die Ribosomenbindungsstelle des L-Peptids. Im kodierenden Bereich schließen sich besonders zweckmäßig die ersten drei Aminosäuren dieses L-Peptids an, worauf dann über eine kurze Aminosäuresequenz die Aminosäuren 23 bis 93 des D-Proteins im trp-Operon folgen.

Die Zwischensequenz Y, die eine Abspaltung des gewünschten Polypeptids ermöglicht, richtet sich nach der Zusammensetzung dieses gewünschten Peptids: Wenn dieses beispielsweise kein Methionin enthält, kann Y Met bedeuten, worauf eine chemische Spaltung mit Bromcyan folgt. Steht im Bindeglied Y am Carboxyterminus Cystein oder steht Y für Cys, so kann eine enzymatische Cystein-spezifische Spaltung oder eine chemische Spaltung, beispielsweise nach spezifischer S-Cyanylierung, folgen. Steht im Brückenglied Y am Carboxyterminus Tryptophan oder steht Y für Trp, so kann eine chemische Spaltung mit N-Bromsuccinimid erfolgen. Steht Y für Asp-Pro, so kann in an sich bekannter Weise (D. Piszkiewicz et al., Biochemical and Biophysical Research Communications 40 (1970) 1173 - 1178) eine proteolytische Spaltung erfolgen. Die Asp-Pro-Bindung kann, wie gefunden wurde, noch säurelabiler gestaltet werden, wenn Y

$(Asp)_m$-Pro bzw. Glu-$(Asp)_m$-Pro

ist, wobei m 1, 2 oder 3 bedeutet. Hierbei werden Spaltprodukte erhalten, die N-terminal mit Pro beginnen bzw. C-terminal mit Asp enden.

Beispiele für enzymatische Spaltungen sind ebenfalls bekannt, wobei auch modifizierte Enzyme mit verbesserter Spezifität eingesetzt werden können (vgl. C.S. Craik et al., Science 228 (1985) 291 - 297). Ist das gewünschte eukaryotische Peptid humanes Proinsulin, so kann man als Sequenz Y eine Peptidsequenz wählen, bei der eine durch Trypsin abspaltbare Aminosäure (Arg, Lys) an die N-terminale Aminosäure (Phe) des Proinsulins gebunden ist, beispielsweise Ala-Ser-Met-Thr-Arg, da dann die Arginin-spezifische Spaltung mit der Protease Trypsin erfolgen kann. Falls das gewünschte Protein nicht die Aminosäurefolge Ile-Glu-Gly-Arg enthält, ist auch eine Spaltung mit Faktor Xa möglich (EP-A-0 161 937).

Bei der Ausgestaltung der Sequenz Y hat man auch die Möglichkeit, auf die synthetischen Gegebenheiten Rücksicht zu nehmen und geeignete Spaltstellen für Restriktionsenzyme einzubauen. Die der Aminosäuresequenz Y entsprechende DNA-Sequenz kann deshalb auch die Funktion eines Linkers oder Adapters übernehmen.

Das erfindungsgemäße Fusionsprotein wird vorteilhaft unter der Kontrolle des trp-Operons von E. coli exprimiert. Ein den Promotor und den Operator des trp-Operons enthaltender DNA-Abschnitt ist inzwischen handelsüblich. Die Expression von Proteinen unter der Kontrolle des trp-Operons ist vielfach beschrieben, beispielsweise in der EP-A2-0 036 776. Die Induktion des trp-Operons kann durch Abwesenheit von L-Tryptophan und/oder Anwesenheit von Indolyl-3-acrylsäure im Medium erreicht werden.

Unter der Kontrolle des trp-Operators erfolgt zunächst die Transkription des L-Peptids, das aus 14 Aminosäuren besteht. Dieses L-Peptid enthält in den Positionen 10 und 11 jeweils L-Tryptophan. Die Geschwindigkeit der Proteinsynthese des L-Peptids bestimmt, ob die nachfolgenden Strukturgene ebenfalls translatiert werden, oder ob die Proteinsynthese abgebrochen wird. Bei Mangel an L-Tryptophan erfolgt nun eine langsame Synthese des L-Peptids infolge einer geringen Konzentration an der tRNA für L-Tryptophan und es erfolgt eine Synthese der folgenden Proteine. Bei hohen Konzentrationen an L-Tryptophan wird dagegen der entsprechende Abschnitt der mRNA rasch abgelesen und es kommt zum Abbruch der Proteinbiosynthese, da die mRNA eine Terminator-ähnliche Struktur annimmt (C. Yanofsky et al., a.a.O.).

Die Häufigkeit der Translation einer mRNA wird stark von der Art der Nukleotide in der Umgebung des Startkodons beeinflußt. Bei der Expression eines Fusionsproteins mit Hilfe des trp-Operons erscheint es daher günstig, die Nukleotide für die ersten Aminosäuren des L-Peptids für den Beginn des Strukturgens des Fusionsproteins einzusetzen. Bei der bevorzugten Ausführung der Erfindung im trp-System wurden die Nukleotide der ersten drei Aminosäuren des L-Peptids (im folgenden L'-Peptid) als Kodons für die N-terminalen Aminosäuren des Fusionsproteins gewählt.

Die Erfindung betrifft deshalb auch Vektoren, vorzugsweise Plasmide zur Expression von Fusionsproteinen, wobei die DNA der Vektoren vom 5'-Ende aus die folgenden Merkmale (in geeigneter Anordnung und in Phase) besitzt: einen Promotor, einen Operator, eine Ribosomen-Bindungsstelle und das Strukturgen für das Fusionsprotein, wobei das letztere die Aminosäuresequenz I (Anhang) vor der Sequenz des gewünschten Proteins enthält. Vor dem Strukturgen bzw. als erstes Triplett des Strukturgens findet sich das Startkodon (ATG) und gegebenenfalls weitere Kodons für andere Aminosäuren, die zwischen dem Start-Kodon und der D'-Sequenz oder zwischen der D'-Sequenz und dem Gen für das gewünschte Protein angeordnet sind. Je nach der Aminosäurezusammensetzung des gewünschten Proteins wird die Auswahl der DNA-Sequenz vor dem Strukturgen getroffen, damit eine Abspaltung des gewünschten Proteins aus dem Fusionsprotein ermöglicht wird.

Bei der Expression des erfindungsgemäßen Fusionsproteins kann es sich als zweckmäßig erweisen, einzelne Tripletts der ersten Aminosäuren nach dem ATG-Startkodon zu verändern, um eine eventuelle Basenpaarung auf der Ebene der mRNA zu verhindern. Solche Veränderungen, ebenso wie Veränderungen, Deletionen oder Additionen einzelner Aminosäuren im D'-Protein sind dem Fachmann geläufig und ebenfalls Gegenstand der Erfindung.

Da kleinere Plasmide mehrere Vorteile bieten, besteht eine bevorzugte Ausgestaltung der Erfindung darin, aus Plasmiden, die sich vom pBR 322 ableiten, einen DNA-Abschnitt mit dem Strukturgen für die Tetracyclinresistenz zu eliminieren. Vorteilhaft entfernt man das Segment von der HindIII-Schnittstelle bei Position 29 bis zur PvuII-Schnittstelle bei Position 2066. Besonders zweckmäßig ist es, bei den erfindungsgemäßen Plasmiden noch einen etwas größeren DNA-Abschnitt zu entfernen, indem man die PvuII-Schnittstelle am Anfang (in Ableserichtung) des trp-Operons nutzt (die in einem nichtessentiellen Teil liegt). Man kann so das entstehende große Fragment mit den beiden PvuII-Schnittstellen direkt ligieren. Das erhaltene, um etwa 2 kbp verkleinerte Plasmid bewirkt eine Expressionssteigerung, die möglicherweise auf eine erhöhte Kopienzahl in der Wirtszelle zurückzuführen ist.

In den folgenden Beispielen wird die Erfindung näher erläutert.

**Beispiel 1**

a) Chromosomale E. coli-DNA wird mit Hinf I geschnitten und das 492 bp-Fragment isoliert, das aus dem trp-Operon den Promotor, Operator, das Strukturgen des L-Peptids, den Attenuator und die Kodons für die ersten sechs Aminosäuren des trp-E-Strukturgens enthält. Dieses Fragment wird mit Hilfe von Klenow-Polymerase mit Desoxynukleotidtriphosphaten aufgefüllt, an beiden Enden mit einem Oligonukleotid verbunden, das eine Erkennungsstelle für Hind III enthält und mit Hind III nachgeschnitten. Das so erhaltene Hind III-Fragment wird in die Hind III-Schnittstelle von pBR 322 ligiert. Man erhält so das Plasmid ptrpE2 - 1 (J.C. Edmann et al., Nature 291 (1981) 503 - 506). Dieses wird wie beschrieben in das Plasmid ptrpL1 überführt.

Mit Hilfe der synthetischen Oligonukleotide (N1) und (N2)

| 5' | CGA | CAA | TGA | AAG | CAA | AGG | 3' | (N1) |
| 5' | CCT | TTG | CTT | TCA | TTG | T | 3' | (N2) |

die zu dem doppelsträngigen Oligonukleotid (N3)

| 5' | CGA | CAA | TGA | AAG | CAA | AGG | 3' | (N3) |
| 3' | T | GTT | ACT | TTC | GTT | TCC | 5' | |

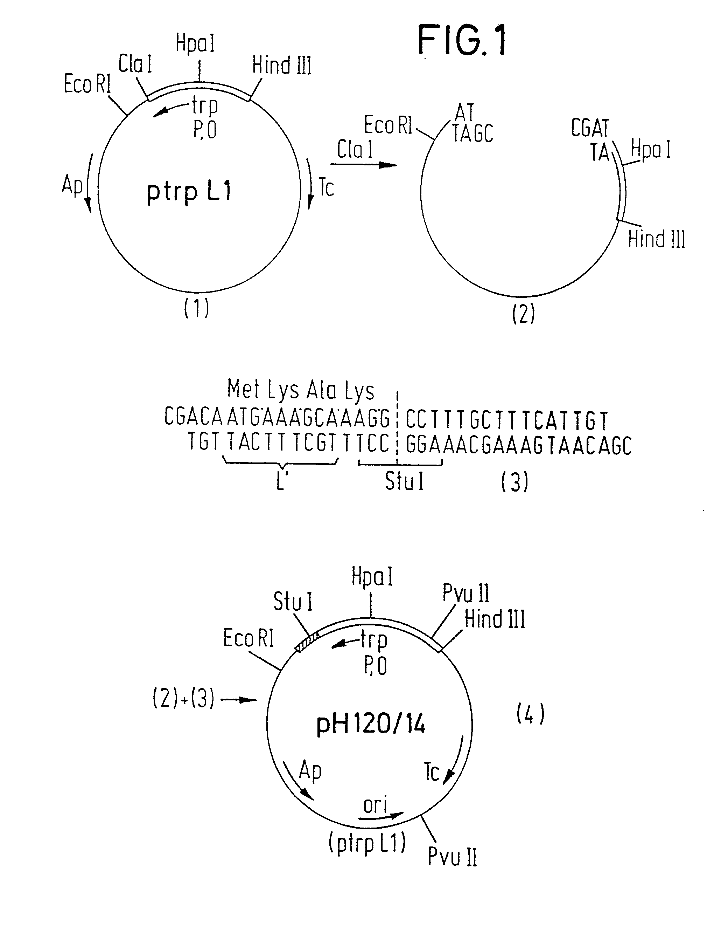

komplementieren, wird in die ClaI-Stelle des Plasmids ptrpL1 die DNA-Sequenz für die ersten drei Aminosäuren des L-Peptids eingebaut sowie eine Restriktionsstelle (StuI) für die Insertion weiterer DNA gebildet (Figur 1). Das Plasmid ptrpL1 wird mit dem Enzym ClaI nach den Angaben des Herstellers umgesetzt, die Mischung mit Phenol extrahiert und die DNA mit Ethanol ausgefällt. Das geöffnete Plasmid wird zur Entfernung der Phosphatgruppen an den 5'-Enden mit Alkalischer Phosphatase aus E. coli umgesetzt. Die synthetischen Nukleotide werden an den 5'-Enden phosphoryliert und mit T4 DNA-Ligase in das geöffnete, Phosphatase-behandelte Plasmid eingesetzt. Nach beendeter Ligase-Reaktion erfolgt die Transformation in E. coli 294 und Selektion der Transformanten durch Amp-Resistenz und Anwesenheit einer StuI-Restriktionsstelle.

Etwa 80 % der erhaltenen Klone wiesen die erwartete Restriktionsstelle auf; die in Figur 1 wiedergegebene Nukleotidsequenz wurde durch Sequenzanalyse bestätigt. Man erhält das Plasmid pH120/14, das nach der RibosomenBindungsstelle für das L-Peptid die Nukleotidtripletts für die ersten drei Aminosäuren des L-Peptids aufweist (L'-Peptid), gefolgt von einer StuI-Stelle, die ihrerseits die Insertion weiterer DNA erlaubt und so die Bildung von Fusionsproteinen mit den ersten drei Aminosäuren des L-Peptids ermöglicht.

b) Im folgenden wird am Beispiel des oben eingesetzten Oligonukleotids (N1) die chemische Synthese solcher Oligonukleotide erläutert:

Nach der Methode von M.J. Gait et al., Nucleic Acids Research 8 (1980) 1081 - 1096, wird das am 3'-Ende stehende Nukleosid, im vorliegenden Falle also Guanosin, kovalent an einen Glaskugel-Träger (CPG = controlled pore glass) LCAA (= long chain alkyl amine) der Fa. Pierce) über die 3'-Hydroxyfunktion gebunden. Hierbei wird das Guanosin als N-2-Isobutyroyl-3'-O-succinoyl5'-dimethoxy-tritylether in Gegenwart von N,N'-Di-cyclohexylcarbodiimid und 4-Dimethylamino-pyridin mit dem modifizierten Träger umgesetzt, wobei die freie Carboxygruppe des Succinoylrestes den Aminorest des langkettigen Amins auf dem Träger acyliert.

In den folgenden Syntheseschritten wird die Basenkomponente als 5'-O-Dimethoxytrityl-nukleosid-3'-phosphorigsäuremonomethylester-dialkylamid oder -chlorid eingesetzt, wobei das Adenin als N6-Benzoyl-Verbindung, das Cytosin als N4-Benzoyl-Verbindung, das Guanin als N2-Isobutyryl-Verbindung und das keine Aminogruppe enthaltende Thymin ohne Schutztruppe vorliegen.

40 mg des Trägers, der 1 µmol Guanosin gebunden enthält, werden nacheinander mit den folgenden Agentien behandelt:

a) Methylenchlorid,
b) 10 % Trichloressigsäure in Methylenchlorid,
c) Methanol,
d) Tetrahydrofuran,
e) Acetonitril,
f) 15 µmol des entsprechenden Nukleosidphosphits und 70 µmol Tetrazol in 0,3 ml wasserfreiem Acetonitril (5 Minuten),
g) 20 % Acetanhydrid in Tetrahydrofuran mit 40 % Lutidin und 10 % Dimethylaminopyridin (2 Minuten),
h) Tetrahydrofuran,
i) Tetrahydrofuran mit 20 % Wasser und 40 % Lutidin,
j) 3 % Jod in Kollidin/Wasser/Tetrahydrofuran im Volumenverhältnis 5 : 4 : 1,
k) Tetrahydrofuran und
l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Diisopropylaminorest, abgesättigt ist. Die Ausbeuten der einzelnen Syntheseschritte können jeweils nach der Detritylierungsreaktion b) spektrophotometrisch durch Messung der Absorption des Dimethoxytritylkations bei einer Wellenlänge von 496 nm bestimmt werden.

Nach abgeschlossener Synthese des Oligonukleotids werden die Methylphosphatschutzgruppen des Oligomers mit Hilfe von p-Thiokresol und Triethylamin abgespalten.

Anschließend wird durch 3-stündige Behandlung mit Ammoniak das Oligonukleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Oligonukleotide synthetisiert.

c) Das Plasmid ptrpED5 - 1 (R.A. Hallewell et al., Gene 9 (1980) 27 - 47) wird mit den Restriktionsenzymen HindIII und SaII nach den Angaben der Hersteller umgesetzt und das etwa 620 bp DNA-Fragment entfernt. Die synthetischen Oligonukleotide (N4) und (N5)

| 5' | AGC | TTC | CAT | GAC | GCG | T | 3' | (N4) |
| 5' | ACG | CGT | CAT | GGA | 3' | | | (N5) |

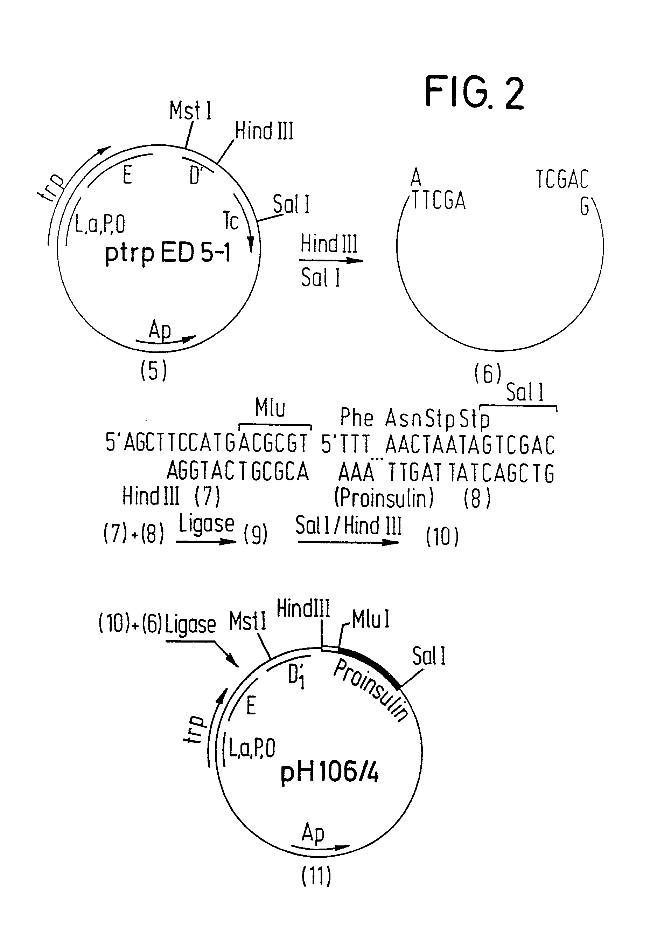

werden phosphoryliert, gemeinsam bei 37°C inkubiert und mit Hilfe von DNA-Ligase an die stumpfendige DNA- für Proinsulin (W. Wetekam et al., Gene 19 (1982) 179 - 183) addiert. Nach Umsetzen mit HindIII und SaII wird die nun verlängerte Proinsulin-DNA mit dem Enzym T4 DNA-Ligase kovalent in das geöffnete Plasmid eingebaut (Figur 2), wobei das Plamid pH106/4 entsteht.

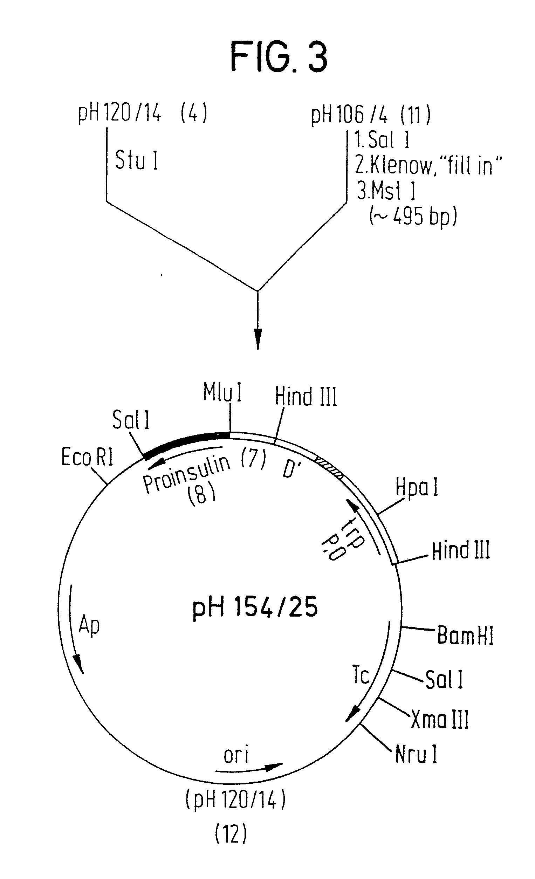

Das Plasmid pH106/4 wird zunächst nochmals mit SaII umgesetzt, die überlappenden Enden mit Klenow-Polymerase in stumpfe Enden ergänzt und anschließend mit dem Enzym MstI inkubiert. Es wird ein etwa 500 bp DNA-Fragment isoliert, das den gesamten kodierenden Teil des Proinsulins sowie einen etwa 210 bp großen Abschnitt des D-Proteins aus dem trp-Operon von E. coli enthält. Das DNA-Fragment ist stumpfendig und wird in die StuI-Stelle des Plasmids pH120/14 inseriert, wobei das Plasmid pH154/25 entsteht (Figur 3). Dieses ist zur Expression eines Fusionsproteins unter der Kontrolle des trp-Operons geeignet, bei dem nach dem L'- und das D'-Peptid die Aminosäuresequenz Ala-Ser-Met-Thr-Arg angeordnet ist, an die sich die Aminosäuresequenz des Proinsulins anschließt.

**Beispiel 2**

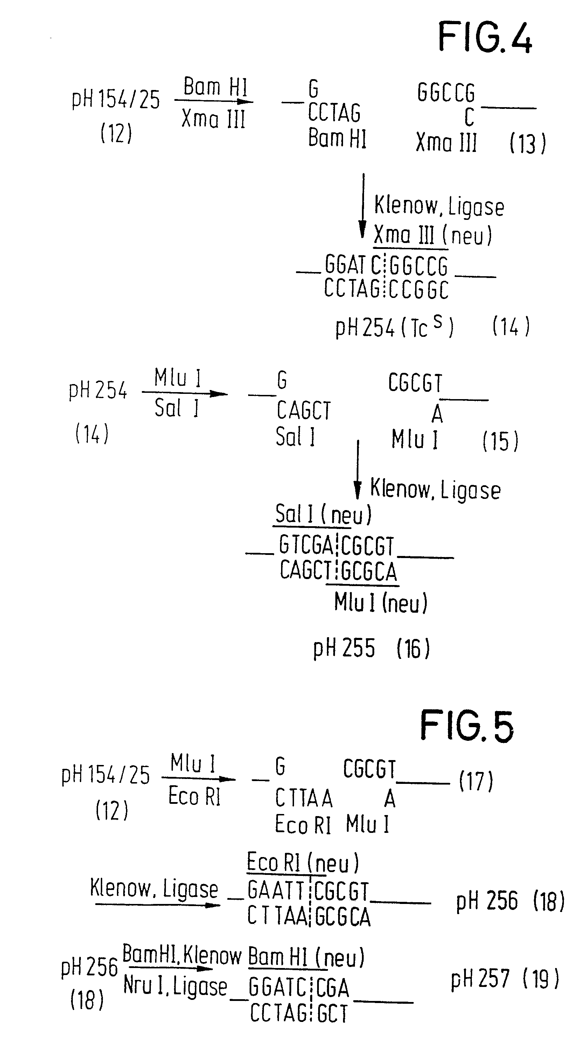

Das Plasmid pH154/25 (Figur 3) wird mit den Restriktionsenzymen BamHI und XmalII umgesetzt. Die überstehenden Enden werden mittels Klenow-Polymerase aufgefüllt und mit T4 DNA-Ligase verknüpft. Man erhält das Plasmid pH254 (Figur 4), das sich zur Expression eines Fusionsproteins mit der Aminosäuresequenz L',D'-Proinsulin unter der Kontrolle des trp-Promotors eignet. Das Plasmid ist etwas kleiner als pH154/25, was von Vorteil sein kann.

**Beispiel 3**

Durch Inkubation des Plasmids pH254 (Beispiel 2; Figur 4) mit den Restriktionsenzymen MluI und SaII wird ein DNA-Abschnitt mit 280 bp freigesetzt, der abgetrennt wird. Das Restplasmid wird mit Klenow-Polymerase in die stumpfendige Form umgewandelt und mit DNA-Ligase wieder kovalent cyclisiert. Dadurch entsteht das Plasmid pH255 (Figur 4), das zur Insertion eines Strukturgens in eine der Restriktionsstellen MluI, SaII und EcoRI geeignet ist. Unter Induktionsbedingungen erfolgt die Bildung Fusionsproteins mit dem L',D'-Protein. Selbstverständlich können durch geeignete Linker weitere Restriktionsstellen in das Plasmid pH255 eingefügt werden.

**Beispiel 4**

Das Plasmid pH154/25 (Figur 3) wird mit den Enzymen MluI und EcoRI inkubiert und das freigesetzte DNA-Fragment (etwa 300 pb) entfernt. Das Restplasmid wird mit Klenow-Polymerase aufgefüllt. Der Ringschluß erfolgt durch Einwirkung von DNA-Ligase. Das entstandene Plasmid pH256 (Figur 5) kann zur Insertion von Strukturgenen in die EcoRI-Stelle verwendet werden.

**Beispiel 5**

Durch Entfernen eines 600 bp-Fragments aus dem Plasmid pH256 (Beispiel 4; Figur 5) mit den Restriktionsenzymen BamHI und NruI erhält man das Plasmid pH257 (Figur 5). Hierzu wird pH256 zunächst mit BamHI inkubiert und stumpfe Enden mit Klenow-Polymerase erzeugt. Nach Inkubation mit NruI und Abtrennung des 600 bp-Fragments erfolgt die Bildung von pH257 nach Inkubation mit DNA-Ligase.

**Beispiel 6**

Durch Insertion des lac-Repressors (P.J. Farabaugh, Nature 274 (1978) 765 - 769) in das Plasmid pKK 177 - 3 (Amann et al. Gene 25 (1983) 167) erhält man das Plasmid pJF118. Dieses wird mit EcoRI und SalI umgesetzt und das Restplasmid isoliert.

Aus dem Plasmid pH106/4 (Figur 2) wird durch Einwirkung von SalI und Inkubation mit MstI ein etwa 495 bp großes Fragment erhalten.

Die synthetisch gewonnenen Oligonukleotide (N6) und (N7)

| | | | | | | | | | |
|---|---|---|---|---|---|---|---|---|---|
| 5' | ACG | AAT | TCA | TGA | AAG | CAA | AGG | 3' | (N6) |
| 5' | CCT | TTG | CTT | TCA | TGA | ATT | CGT | 3' | (N7) |

werden phosphoryliert und mit DNA-Ligase an das stumpfendige DNA-Fragment addiert. Durch Umsetzung mit EcoRI und SalI werden überlappende Enden freigesetzt, die eine Ligierung in das geöffnete Plasmid pJF118 erlauben.

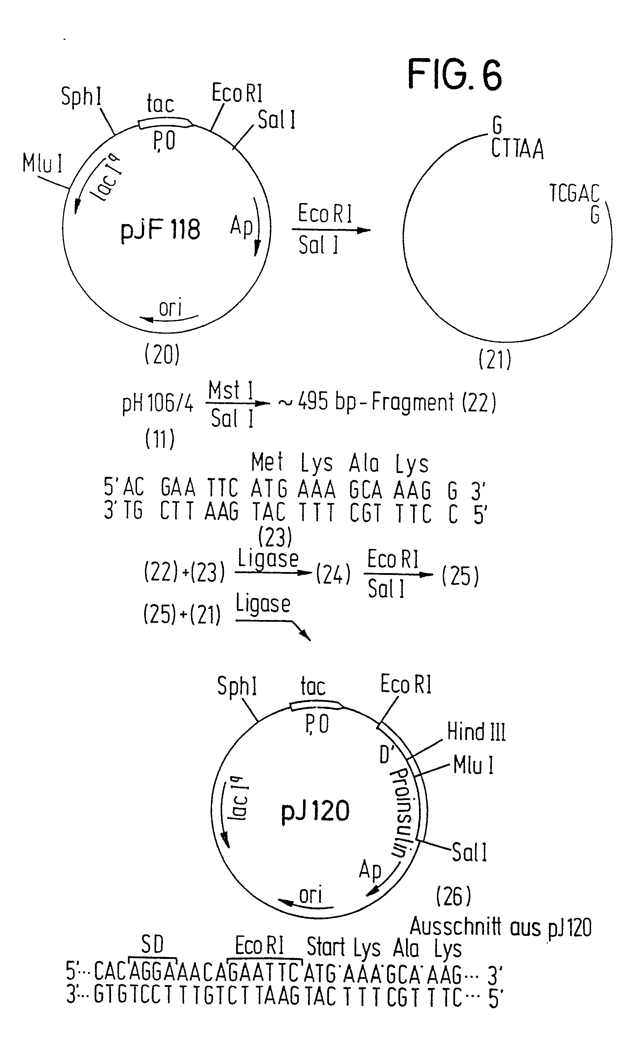

Nach Transformation des so erhaltenen Hybridplasmids in E. coli 294 werden aufgrund der Größe der Restriktionsfragmente die richtigen Klone ausgewählt. Dieses Plasmid wird als pJ120 bezeichnet (Figur 6).

Die Expression des Fusionsproteins wird im Schüttelkolben wie folgt durchgeführt:

Aus einer Übernachtkultur von E. coli 294-Transformanten, die das Plasmid pJ120 enthalten, in LB-Medium (J.H. Miller, Experiments in Molecular Genetics, Cold Spring Harbor Laboratory, 1972) mit 50 µg/ml Ampicillin wird eine frische Kultur im Verhältnis von etwa 1 : 100 angesetzt und das Wachstum über OD-Messung verfolgt. Bei OD = 0,5 wird die Kultur mit soviel Isopropyl-β-D-galactopyranosid (IPTG) versetzt, daß dessen Konzentration 1 mM beträgt, und die Bakterien nach 150 bis 180 Minuten abzentrifugiert. Die Bakterien werden 5 Minuten in einer Puffermischung (7M Harnstoff, 0,1 % SDS, 0,1 M Natriumphosphat, pH 7,0) gekocht und Proben auf eine SDS-Gelelektrophoreseplatte aufgetragen. Nach Elektrophorese wird aus Bakterien, die das Plasmid pJ120 enthalten, eine Proteinbande erhalten, die der Größe des erwarteten Fusionsproteins entspricht und mit Antikörpern gegen Insulin reagiert. Nach Isolierung des Fusionsproteins kann man durch BromcyanSpaltung das erwartete Proinsulin-Derivat freisetzen. Nach Aufschluß der Bakterien (French Press; [R]Dyno-Mühle) und Zentrifugation befindet sich das L',D'-Proinsulin-Fusionsprotein im Niederschlag, so daß mit dem Überstand bereits erhebliche Mengen der übrigen Proteine abgetrennt werden können.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

**Beispiel 7**

Aus E. coli 294-Transformanten, die das Plasmid pH154/25 (Figur 3) enthalten, wird eine Übernachtkultur in LB-Medium mit 50 µg/l Ampicillin hergestellt und am nächsten Morgen im Verhältnis von etwa 1 : 100 in M9-Medium (J.H. Miller, a.a.O.) mit 2000 µg/ml Casamino Acids und 1 µg/ml Thiamin verdünnt. Bei OD = 0,5 wird Indolyl-3-acrylsäure zugesetzt, so daß die Endkonzentration 15 µg/ml beträgt. Nach 2 bis 3 Stunden Inkubation werden die Bakterien abzentrifugiert. Eine SDS-Gelelektrophorese zeigt an der für das Fusionsprotein erwarteten Stelle eine sehr deutliche Proteinbande, die mit Antikörpern gegen Insulin reagiert. Nach Aufschluß der Bakterien und Zentrifugation befindet sich das L',D'-Proinsulin-Fusionsprotein im Niederschlag, so daß auch hier mit dem Überstand bereits erhebliche Mengen der übrigen Proteine abgetrennt werden.

Auch im vorliegenden Falle gelten die angegebenen Induktionsbedingungen für Schüttelkulturen. Fermentationen in größerem Volumen erfordern veränderte Konzentrationen an Casamino Acids bzw. einen Zusatz an L-Tryptophan.

**Beispiel 8**

Das Plasmid pH154/25 (Figur 3) wird mit EcoRI geöffnet und die überstehenden DNA-Einzelstränge mit Klenow-Polymerase aufgefüllt. Die so erhaltene DNA wird mit dem Enzym MluI inkubiert und die für Insulin kodierende DNA aus dem Plasmid herausgeschnitten. Durch gelelektrophoretische Auftrennung wird dieses Fragment vom Restplasmid abgetrennt und das Restplasmid isoliert.

Das Plasmid gemäß Figur 3 der deutschen Offenlegungsschrift 3 429 430 (Europäische Patentanmeldung EP-A1-0 171 024) wird mit den Restriktionsenzymen AccI und SalI umgesetzt und das die Hirudinsequenz enthaltende DNA-Fragment abgetrennt. Nach Auffüllen der überstehenden Enden der SalI-Schnittstelle mit Klenow-Polymerase wird das DNA-Segment mit der synthetischen DNA der Formel (N8)

Met Thr

| | | | | | | | | |
|---|---|---|---|---|---|---|---|---|
| 5' | CCC | ACG | CGT | ATG | ACG | T | 3' | |
| 3' | GGG | TGC | GCA | TAC | TGC | ATA | 5' | (N8) |

ligiert. Das Ligationsprodukt wird mit MluI inkubiert. Nach Inaktivierung des Enzyms bei 65°C wird das DNA-Gemisch

6

# EP 0 211 299 B1

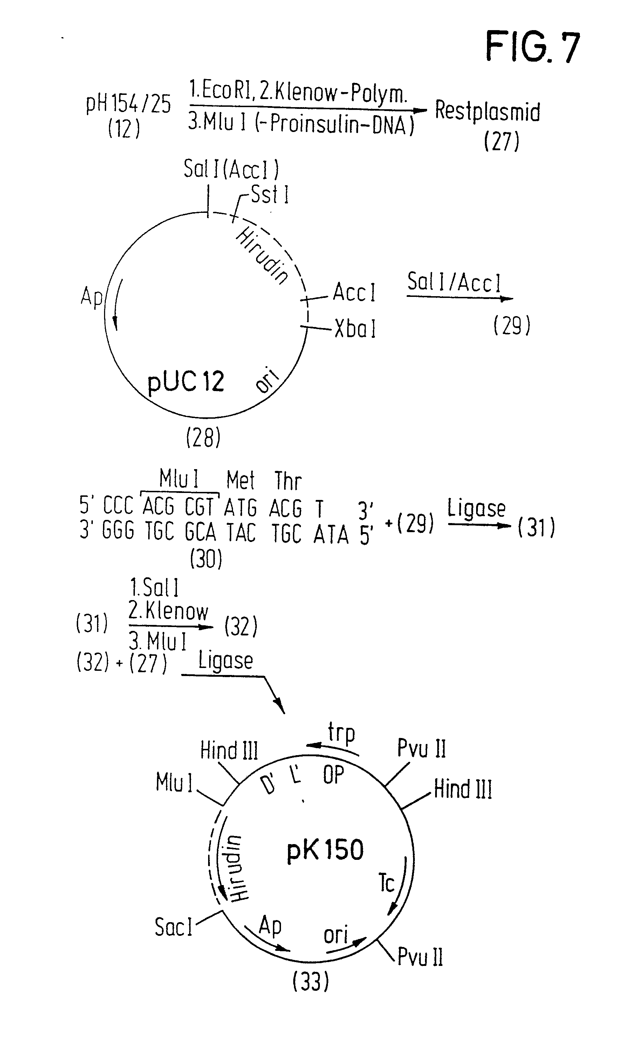

mit Alkalischer Rinderphosphatase eine Stunde bei 37°C behandelt. Hierauf werden die Phosphatase und das Restriktionsenzym mittels Phenolextraktion aus dem Gemisch entfernt und die DNA durch Ethanolfällung gereinigt. Die so behandelte DNA wird mit T4-Ligase in das geöffnete Restplasmid pH154/25 eingesetzt, wobei das Plasmid pK150 erhalten wird, das durch Restriktionsanalyse und DNA-Sequenzierung nach Maxam und Gilbert charakterisiert wurde (Figur 7).

## Beispiel 9

E. coli 214-Bakterien, die das Plasmid pK150 (Figur 7) enthalten, werden in LB-Medium mit 30 bis 50 µg/ml Ampicillin über Nacht bei 37°C angezogen. Die Kultur wird mit M9-Medium, 2000 µg/l Casamino Acids und 1 µg/l Thiamin enthält, im Verhältnis 1 : 100 verdünnt und bei 37°C unter ständiger Durchmischung inkubiert. Bei einer $OD_{600}$ = 0,5 bzw. 1 wird bis zu einer Endkonzentration von 15 µg/l Indolyl-3-acrylsäure zugesetzt und 2 bis 3 Stunden bzw. 16 Stunden inkubiert. Anschließend werden die Bakterien abzentrifugiert und in 0,1 M Natriumphosphatpuffer (pH 6,5) unter Druck aufgeschlossen. Die schwerlöslichen Proteine werden abzentrifugiert und durch SDS-Polyacrylamid-Elektrophorese analysiert. Es zeigt sich, daß Zellen, deren trp-Operon induziert wurde, im Bereich unterhalb 20 000 Dalton, aber größer als 14 000 Dalton, ein neues Protein enthalten, das in nicht induzierten Zellen nicht gefunden wird. Nach Isolation des Fusionsproteins und Umsetzung mit Bromcyan wird Hirudin freigesetzt.

## Beispiel 10

Im folgenden werden Konstruktionen beschrieben, die es erlauben, vor das 5'-Ende der trp-D-Sequenz DNA-Sequenzen einzuführen, die möglichst zahlreiche Erkennungsstellen für verschiedene Restriktionsenzyme enthalten, um die trp-D-Sequenz möglichst universell in die verschiedensten prokaryotischen Expressionssysteme einbauen zu können.

Die Plasmide pUC12 und pUC13 (Pharmacia P-L Biochemical 5401 St. Goar: The Molecular Biology Catalogue 1983, Appendix, p. 89) enthalten eine Polylinkersequenz wobei im Plasmid pUC13 zwischen den Restriktionsschnittstellen für XmaI und SacI das MstI-Hind III-trp-Fragment aus dem Plasmid p106/4 (Figur 2), fusioniert mit dem Hind III-Hirudin-SacI-Fragment aus dem Plasmid pK150 (Figur 7) eingesetzt werden soll.

Hierzu wird die DNA des Plasmids pUC13 zunächst mit dem Restriktionsenzym XmaI behandelt. Die Enden des linearisierten Plasmids werden mittels Klenow-Polymerase-Reaktion aufgefüllt. Nach Ethanolfällung wird die DNA mit dem Enzym SacI behandelt und mit Ethanol erneut aus dem Reaktionsgemisch gefällt. Die DNA wird nun in einem wäßrigen Ligationsansatz mit dem MstI-Hind III-trp-D-Fragment, isoliert aus Plasmid pH106/4, und dem Hind III-SacI-Hirudin-Fragment, isoliert aus dem Plasmid pK150, und T4 DNA-Ligase umgesetzt.

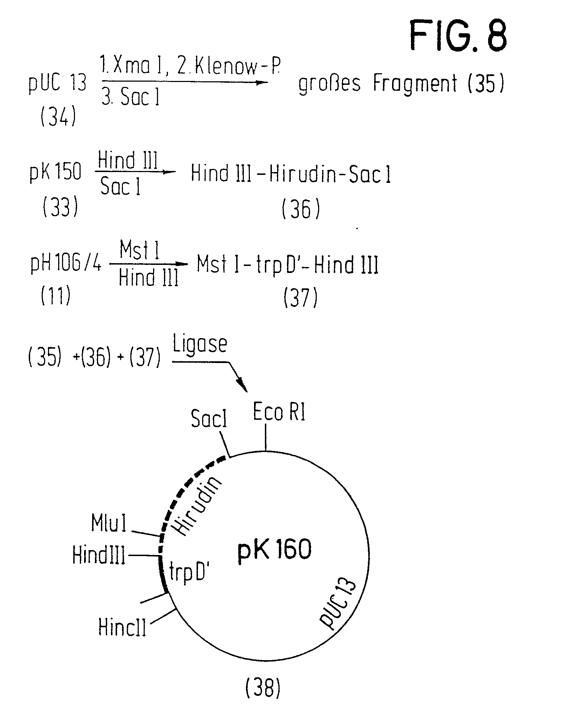

Das so erhaltene Plasmid pK160 enthält nun unmittelbar vor Beginn der trp-D-Sequenz eine multible Restriktionsenzym-Erkennungssequenz, die Schnittstellen für die Enzyme XmaI, SmaI, BamHI, XbaI, Hind II, SalI, AccI, PstI und Hind III umfaßt. Durch diese Konstruktion wird ferner nach dem 3'-Ende der Hirudin-Sequenz eine EcoRI-Schnittstelle erzeugt (Figur 8).

## Beispiel 11

Das Plasmid pH131/5 wird (gemäß der nicht veröffentlichten deutschen Patentanmeldung P-3 514 113.1, Beispiel 1, Figur 1) wie folgt hergestellt:

Das Plasmid ptrpL1 (J.C. Edman et al., Nature 291 (1981) 503 - 506) wird mit ClaI geöffnet und mit dem synthetisch hergestellten, selbst-komplementären Oligonucleotid (N9)

5'     pCGACCATGGT 3'; (N9)

ligiert.

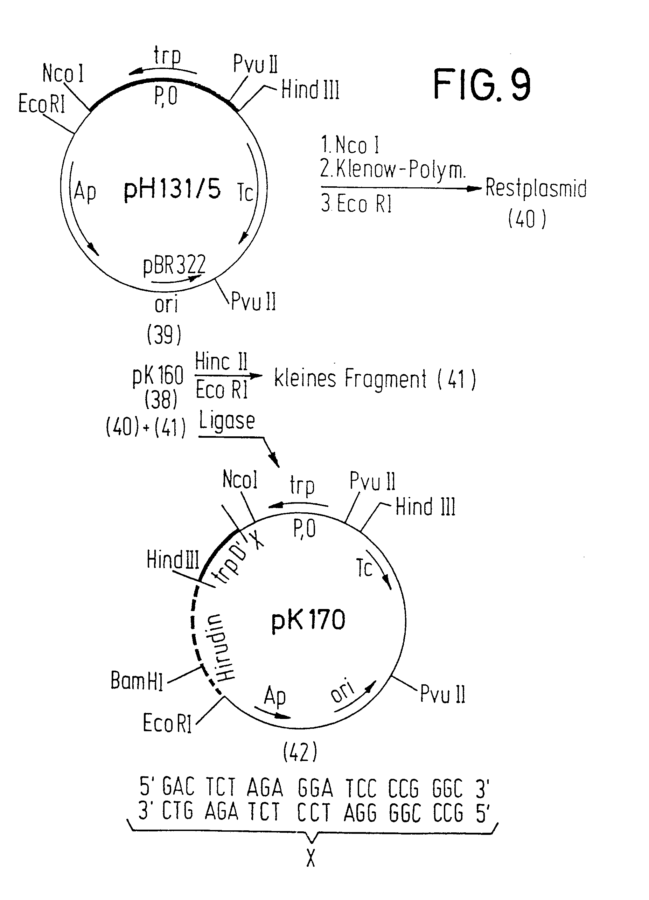

Das so erhaltene Plasmid pH131/5 (Figur 9) wird an der derart eingeführten Schnittstelle für das Restriktionsenzym NcoI geöffnet und die entstandenen überstehenden Einzelstrangenden mittels Klenow-Polymerase-Reaktion aufgefüllt. Die linearisierte glattendige DNA wird nun mit dem Enzym EcoRI nachgeschnitten und die größere der beiden entstandenen DNA-Sequenzen mittels Ethanolfällung von der kleineren Sequenz abgetrennt. Die so gewonnene Restplasmid-DNA des Plasmids pH131/5 wird nun mit einem für trp-D'-Hirudin kodierenden Fragment aus pK160 durch T4-Ligase-Reaktion ligiert. Dieses Fragment wurde durch Öffnen des Plasmids mit HincII und EcoRI aus dem Plasmid pK160 herausgespalten. Das Fragment wird gelelektrophoretisch vom Restplasmid abgetrennt und anschließend aus dem Gelmaterial eluiert. Das Ligationsprodukt wird nach E. coli K12 transformiert. Die Plasmid-DNA enthaltenden Klone werden isoliert und durch Restriktionsanalyse und DNA-Sequenz-Analyse charakterisiert. Das so erhaltene Plasmid pK170 enthält an den trp-Operator fusioniert eine DNA-Sequenz, die für Met-Asp-Ser-Arg-Gly-Ser-Pro-Gly-trp-D'-(Hirudin) kodiert (Figur 9).

7

**Beispiel 12**

Das Plasmid pJF118 (Beispiel 6) wird mit EcoRI geöffnet und die überstehenden DNA-Enden mittels Klenow-Polymerase-Reaktion in glatte Enden überführt. Die so behandelte DNA wird anschließend mit dem Enzym SalI geschnitten und gelelektrophoretisch von dem kurzen EcoRI SalI-Fragment abgetrennt.

Das Plasmid pK 170 (Beispiel 11) wird mit NcoI gespalten und die überstehenden Enden mittels Klenow-Polymerase in glatte Enden überführt. Die Plasmid-DNA wird aus dem Reaktionsgemisch durch Ethanolfällung abgetrennt und mit den Enzymen Hind III und BamHI behandelt. Von den entstehenden Fragmenten werden zwei isoliert, nämlich das NcoI (mit aufgefülltem Ende)-trpD'-Hind III-Fragment und das Hind III-Hirudin-BamHI-Fragment (deutsche Offenlegungsschrift 3 429 430). Beide Fragmente werden nach gelelektrophoretischer Auftrennung isoliert.

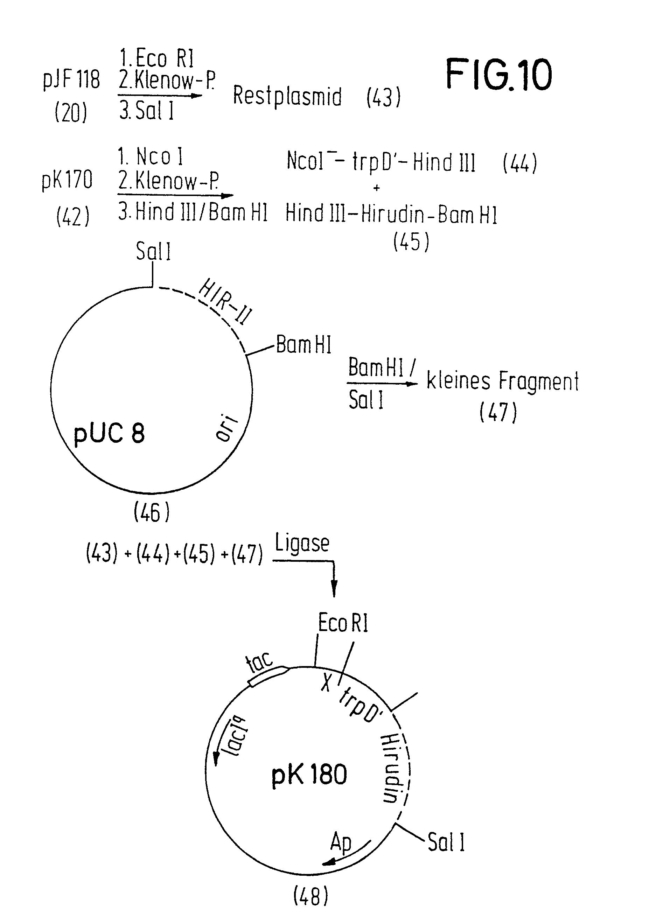

Ferner wird das BamHI-SalI-Hirudin II-Fragment gemäß Figur 2 der deutschen Offenlegungsschrift 3 429 430 isoliert. In einer Ligationsreaktion werden nun die vier Fragmente, nämlich das pJF 118-Restplasmid, das NcoI-trpD'-Hind III-Fragment, das Hind III-Hirudin-BamHI-Fragment und das Hirudin II-Fragment, miteinander umgesetzt und das erhaltene Plasmid pK 180 (Figur 10) nach E. coli K12-W 3110 transformiert. Richtige Plasmide sind dadurch gegeben, daß ein EcoRI-trpD'-Hirudin-SalI-Fragment in der Plasmid-DNA nachgewiesen werden kann. Die trpD'-Hirudin-Sequenz ist nun an den tac-Promotor angeschlossen. Das Fusionsprotein wird gemäß Beispiel 6 exprimiert.

**Beispiel 13**

Bei den Plasmiden, die sich von pBR 322 ableiten, wie pH120/14 (Beispiel 1, Figur 1), pH154/25 (Beispiel 1, Figur 3), pH256 (Beispiel 4, Figur 5), pK150 (Beispiel 8, Figur 7) und pK170 (Beispiel 11, Figur 9) befindet sich - auf den Figuren im Uhrzeigersinn - zwischen dem Startcodon des Fusionsproteins und der nächsten Hind III-Stelle (entsprechend Hind III bei Position 29 in pBR322) eine zusätzliche PvuII-Stelle im Bereich des Fragments, das den trp-Promotor und -Operator enthält, allerdings außerhalb des Promotor-Bereichs.

Es wurde nun festgestellt, daß durch Entfernen des DNA-Abschnittes, der durch die beschriebene PvuII-Stelle und die PvuII-Stelle, die der Position 2066 in pBR322 entspricht, begrenzt wird, die Ausbeute eines klonierten Proteins (oder Fusionsproteins) deutlich erhöht wird. Die verkürzten Plasmid werden durch einen Stern gekennzeichnet.

Im folgenden wird beispielhaft die Verkürzung des Plasmids pH154/25 zu pH154/25* beschrieben, die für die übrigen obengenannten Plasmide entsprechend erfolgen kann:

pH154/25 wird (nach den Angaben des Herstellers) mit PvuII umgesetzt, wobei drei Fragmente entstehen:

Fragment 1: Von der PvuII-Restriktionsstelle des Proinsulins bis zu der der Position 2066 in pBR322 entsprechenden PvuII-Restriktionsstelle,

Fragment 2: von der PvuII-Restriktionsstelle nahe dem trp-Promotor bis zur PvuII-Stelle des Proinsulins und

Fragment 3: von der PvuII-Stelle nahe dem trp-PromotorFragmentes bis zu der der Position 2066 in pBR322 entsprechenden PvuII-Stelle.

Die Fragmente können durch Elektrophorese auf Agarose getrennt und anschließend isoliert werden (Maniatis, a.a.O.).

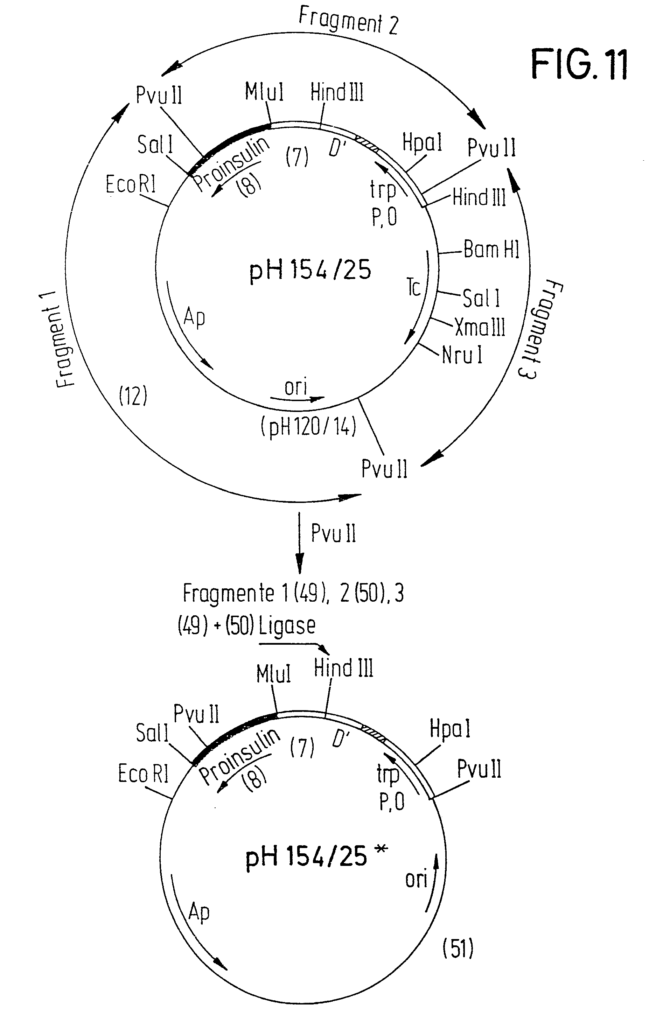

Die Fragmente 1 und 2 werden unter "blunt end"-Bedingungen mit dem Enzym T4 DNA-Ligase verbunden. Nach Transformation in E. coli 294 wird geprüft, in welchen Kolonien ein Plasmid mit der vollständigen Proinsulinsequenz vorhanden ist und somit die Fragmente in der gewünschten Anordnung vorliegen. Das Plasmid pH154/25* ist in der Figur 11 dargestellt.

Bei der Expression, die wie in den vorstehenden Beispielen beschrieben erfolgt, wird eine deutliche Steigerung des Fusionsproteinanteils beobachtet.

**Beispiel 14**

Das Plasmid pH 154/25* (Beispiel 13, Figur 11) wird mit Hind III und SalI verdaut und das kleine Fragment (mit der Proinsulinsequenz) gelelektrophoretisch abgetrennt. Das große Fragment wird isoliert und mit der synthetischen DNA (N10)

|   | (Ala) | Trp | Glu | Asp | Pro | Met | Ile | Glu | (Gly) | (Arg) |   |
|---|-------|-----|-----|-----|-----|-----|-----|-----|-------|-------|---|
| A | GCT   | TGG | GAG | GAT | CCT | ATG | ATC | GAG | GG    |       | (N10) |
|   |       | ACC | CTC | CTA | GGA | TAC | TAG | CTC | CCA   | GCT   |   |

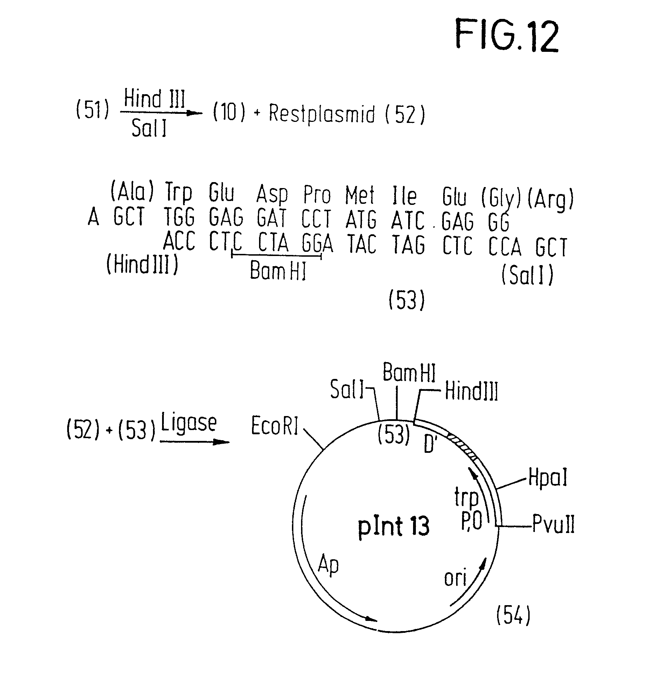

ligiert. Es entsteht das Plasmid pInt13 (Figur 12).

Die DNA (N10) kodiert für eine Aminosäuresequenz, die mehrere Spaltstellen für eine chemische Spaltung enthält:

a) Met für Bromcyan,
b) Trp für N-Bromsuccinimid (NBS oder BSI)
c) Asp-Pro für proteolytische Spaltung, wobei das vorangestellte Glu die Asp-Pro-Bindung zusätzlich gegenüber der Einwirkung von Säuren schwächt.

Die Einführung dieses HindIII-SalI-Linkers (N10) in den Leserahmen eines kodierten Polypeptids erlaubt also die angeführten Möglichkeiten für eine chemische Abspaltung, je nach der Aminosäurefolge des erwünschten Proteins bzw. nach seiner Empfindlichkeit gegenüber den Spaltungsagenzien.
Die Figuren sind nicht maßstabgerecht.

**Aminosäuresequenz I**

23
Ser  Asn  Gly  His  Asn  Val  Val  Ile

      10
Tyr  Arg  Asn  His  Ile  Pro  Ala  Gln

                  20
Thr  Leu  Ile  Glu  Arg  Leu  Ala  Thr

                                30
Met  Ser  Asn  Pro  Val  Leu  Met  Leu

                                        40
Ser  Pro  Gly  Pro  Gly  Val  Pro  Ser

Glu  Ala  Gly  Cys  Met  Pro  Glu  Leu

      50
Leu  Thr  Arg  Leu  Arg  Gly  Lys  Leu

                  60
Pro  Ile  Ile  Gly  Ile  Cys  Leu  Gly

                              70   93
His  Gln  Ala  Ile  Val  Glu  Ala

**DNA-Sequenz I**

Ser  Asn  Gly  His  Asn  Val  Val  Ile
AGC  AAT  GGG  CAT  AAC  GTG  GTG  ATT

      10
Tyr  Arg  Asn  His  Ile  Pro  Ala  Gln
TAC  CGC  AAC  CAT  ATA  CCG  GCG  CAA

                  20
Thr  Leu  Ile  Glu  Arg  Leu  Ala  Thr
ACC  TTA  ATT  GAA  CGC  TTG  GCG  ACC
50

                                30
Met  Ser  Asn  Pro  Val  Leu  Met  Leu
ATG  AGT  AAT  CCG  GTG  CTG  ATG  CTT

                                        40
Ser  Pro  Gly  Pro  Gly  Val  Pro  Ser
TCT  CCT  GGC  CCC  GGT  GTG  CCG  AGC
      100

Glu  Ala  Gly  Cys  Met  Pro  Glu  Leu
GAA  GCC  GGT  TGT  ATG  CCG  GAA  CTC

|  | 50 |  |  |  |  |  |  |
|---|---|---|---|---|---|---|---|
| Leu | Thr | Arg | Leu | Arg | Gly | Lys | Leu |
| CTC | ACC | CGC | TTG | CGT | GGC | AAG | CTG |
|  | 150 |  |  |  |  |  |  |

|  |  |  | 60 |  |  |  |  |
|---|---|---|---|---|---|---|---|
| Pro | Ile | Ile | Gly | Ile | Cys | Leu | Gly |
| CCC | ATT | ATT | GGC | ATT | TGC | CTC | GGA |

|  |  |  |  |  | 70 |  |
|---|---|---|---|---|---|---|
| His | Gln | Ala | Ile | Val | Glu | Ala |
| Cat | CAG | GCG | ATT | GTC | GAA | GCT |
|  |  | 200 |  |  |  |  |

**Patentansprüche** für die Vertragsstaaten: BE, CH, DE, FR, GB, IT, LI, LU, NL, SE

1. Fusionsprotein der allgemeinen Formel

Met - $X_n$ - D' - Y - Z

in der

n  Null oder 1 ist,
X  eine Sequenz von 1 bis 12 genetisch kodierbaren Aminosäuren ist,
D'  eine Sequenz von etwa 70 Aminosäuren im Bereich der Aminosäuresequenz 23 - 93 des D-Peptids im trp Operon von E. coli ist,
Y  eine Sequenz aus einer oder mehreren genetisch kodierbaren Aminosäuren bedeutet, die eine Abspaltung der folgenden Aminosäuresequenz Z ermöglicht, und
Z  eine Sequenz aus genetisch kodierbaren Aminosäuren ist.

2. Fusionsprotein nach Anspruch 1, dadurch gekennzeichnet, daß n eins ist und X bis zu 5 Aminosäuren umfaßt, wobei vorzugsweise in X N-terminal Lys-Ala steht.

3. Fusionsprotein nach Anspruch 1 oder 2, dadurch gekennzeichnet, daß Y C-terminal Met, Cys, Trp, Arg oder Lys oder eine der Gruppen

(Asp)$_m$-Pro bzw. Glu-(Asp)$_m$-Pro oder Ile-Glu-Gly-Arg

in denen m 1, 2 oder 3 bedeutet,
enthält oder aus diesen Aminosäuren oder einer dieser Gruppen besteht.

4. Fusionsprotein nach einem oder mehreren der vorhergehenden Ansprüche, dadurch gekennzeichnet, daß Z die Aminosäuresequenz von humanem Proinsulin oder einem Hirudin bedeutet.

5. Verfahren zur Herstellung der Fusionsproteine nach einem oder mehreren der Ansprüche 1 bis 4, dadurch gekennzeichnet, daß man eine für diese Fusionsproteine kodierende Genstruktur in einer Wirtszelle, vorzugsweise in einem Bakterium, insbesondere E. coli, exprimiert und das Fusionsprotein abtrennt.

6. Verfahren nach Anspruch 5, dadurch gekennzeichnet, daß in der Genstruktur für D' die DNA-Sequenz I (Anhang) kodiert, und/oder in der Genstruktur für X die DNA-Sequenz (kodierender Strang)

5'     AAA     GCA     AAG     GGC     3'

kodiert, und/oder die Genstruktur so gewählt wird, daß das Fusionsprotein unlöslich ist, und/oder die Genstruktur in Phase in einem Vektor enthalten ist, der aus dem trp-Operon von E. coli den Promotor, den Operator und die Ribosomenbindungsstelle des L-Peptids enthält.

7. Verfahren nach Anspruch 6, dadurch gekennzeichnet, daß der Vektor ein Derivat von pBR322 ist, wobei aus der pBR322-DNA der Abschnitt von der Hind III-Stelle bei Position 29 bis zur PvuII-Stelle bei Position 2066 entfernt ist.

8. Genstruktur, kodierend für Fusionsproteine nach Anspruch 1 bis 4.

9. Vektor, vorzugsweise Derivat des Plasmids pBR322, wobei aus der pBR322-DNA der Abschnitt von der Hind III-Stelle bei Position 29 bis zur PvuII-Stelle bei Position 2066 entfernt ist, enthaltend eine Genstruktur nach Anspruch 8.

10. Expressionssystem, vorzugsweise E. coli-System, enthaltend einen Vektor nach Anspruch 9.

11. Verfahren zur Herstellung eines eukaryotischen Proteins, dadurch gekennzeichnet, daß man aus einem Fusionsprotein nach Anspruch 1 bis 4 die Aminosäuresequenz Z chemisch oder enzymatisch abspaltet.

12. Plasmide pH 154/25 (Fig. 3), pH 254 (Fig. 4), pH 255 (Fig. 4), pH 256 (Fig. 5), pH 257 (Fig. 5), pH 120/14, pK 150 (Fig. 7), pK 160, (Fig. 8), pK 170 (Fig. 9), pK 180 (Fig. 10), pH 154/25* (Fig. 11), pH 256*, pH 120/14*, pK 150*, pK 170* und pInt 13 (Fig. 12).

**Patentansprüche** für den Vertragsstaat: AT

1. Verfahren zur Herstellung eines Fusionsproteins der allgemeinen Formel (1)

$$\text{Met-X}_n\text{-D'-Y-Z} \qquad\qquad (1)$$

in der

n  Null oder 1 ist,
X  eine Sequenz von 1 bis 12 genetisch kodierbaren Aminosäuren ist,
D'  eine Sequenz von etwa 70 Aminosäuren im Bereich der Aminosäuresequenz 23 - 93 des D-Peptids im trp Operon von E. coli ist,
Y  eine Sequenz aus einer oder mehreren genetisch kodierbaren Aminosäuren bedeutet, die eine Abspaltung der folgenden Aminosäuresequenz Z ermöglicht, und
Z  eine Sequenz aus genetisch kodierbaren Aminosäuren ist, dadurch gekennzeichnet, daß man eine für diese Fusionsproteine kodierende Genstruktur in einer Wirtszelle exprimiert und das Fusionsprotein abtrennt.

2. Verfahren nach Anspruch 1, dadurch gekennzeichnet, daß man eine Genstruktur für ein Fusionsprotein der Formel 1 einsetzt, in der n eins ist und X bis zu 5 Aminosäuren umfaßt.

3. Verfahren nach Anspruch 1 oder 2, dadurch gekennzeichnet, daß man eine Genstruktur für ein Fusionsprotein der Formel 1 einsetzt, in der n eins ist und in X N-terminal Lys-Ala steht.

4. Verfahren nach einem oder mehreren der vorhergehenden Ansprüche, dadurch gekennzeichnet, daß man eine Genstruktur für ein Fusionsprotein der Formel 1 einsetzt, in der Y C-terminal Met, Cys, Trp, Arg oder Lys oder eine der Gruppen $(\text{Asp})_m$-Pro bzw. Glu-(Asp) -Pro oder Ile-Glu-Gly-Arg, in denen m 1, 2 oder 3 bedeutet, enthält oder aus diesen Aminosäuren besteht.

5. Verfahren nach einem oder mehreren der vorhergehenden Ansprüche, dadurch gekennzeichnet, daß man eine Genstruktur für ein Fusionsprotein der Formel 1 einsetzt, in der Z die Aminosäuresequenz von humanem Proinsulin oder einem Hirudin bedeutet.

6. Verfahren nach einem oder mehreren der vorhergehenden Ansprüche, dadurch gekennzeichnet, daß man eine Genstruktur für ein Fusionsprotein der Formel 1 einsetzt, in der für D' die DNA-Sequenz I (Anhang) kodiert.

7. Verfahren nach Anspruch 6, dadurch gekennzeichnet, daß in der Genstruktur für X die DNA-Sequenz (kodierender Strang)

5'    AAA    GCA    AAG    GGC    3'

kodiert.

8. Verfahren nach einem oder mehreren der vorhergehenden Ansprüche, dadurch gekennzeichnet, daß die Genstruktur so gewählt wird, daß das Fusionsprotein unlöslich ist.

9. Verfahren nach einem oder mehreren der vorhergehenden Ansprüche, dadurch gekennzeichnet, daß die Genstruktur in Phase in einem Vektor enthalten ist, der aus dem trp-Operon von E. coli den Promotor, den Operator und die Ribosomenbindungsstelle des L-Peptids enthält.

10. Verfahren nach Anspruch 9, dadurch gekennzeichnet, daß der Vektor ein Derivat von pBR322 ist, wobei aus der pBR322-DNA der Abschnitt von der Hind III-Stelle bei Position 29 bis zur PvuII-Stelle bei Position 2066 entfernt ist.

11. Verfahren nach einem oder mehreren der vorhergehenden Ansprüche, dadurch gekennzeichnet, daß die Wirtszelle E. coli ist.

12. Verfahren zur Herstellung eines eukaryotischen Proteins, dadurch gekennzeichnet, daß man aus einem nach Anspruch 1 bis 5 erhaltenen Fusionsprotein die Aminosäuresequenz Z chemisch oder enzymatisch abspaltet.

# EP 0 211 299 B1

**Revendications** Pour les Etats Contractants: BE, CH, DE, FR, GB, IT, LI, NL, SE

1. Protéine de fusion de formule générale

$Met - X_n - D' - Y - Z$

dans laquelle

n est zéro ou 1,

X est une séquence de 1 à 12 aminoacides génétiquement codables,

D' est une séquence d'environ 70 aminoacides dans la région de la séquence d'aminoacides 23 - 93 du peptide D dans l'opéron trp de *E. coli*,

Y représente une séquence constituée d'un ou plusieurs aminoacides génétiquement codables, qui permet une séparation de la séquence d'aminoacides Z suivante, et

Z est une séquence d'aminoacides génétiquement codables.

2. Protéine de fusion selon la revendication 1, caractérisée en ce que n est 1 et X comprend jusqu'à 5 aminoacides, Lys-Ala se trouvant de préférence à l'extrémité N-terminale dans X.

3. Protéine de fusion selon la revendication 1 ou 2, caractérisée en ce que Y contient à l'extrémité C-terminale Met, Cys, Trp, Arg ou lys, ou l'un des groupes $(Asp)_m$-Pro ou Glu-$(Asp)_m$-Pro ou Ile-Glu-Gly-Arg, dans lesquels m représente 1, 2 ou 3, ou consiste en ces acides aminés ou en un de ces groupes.

4. Protéine de fusion selon une ou plusieurs des revendications précédentes, caractérisée en ce que Z représente la séquence d'aminoacides de la proinsuline humaine ou d'une hirudine.

5. Procédé pour la préparation des protéines de fusion selon une ou plusieurs des revendications 1 à 4, caractérisé en ce que l'on exprime une structure génique codant pour cette protéine de fusion dans une cellule hôte, de préférence dans une bactérie, en particulier *E. coli*, puis on sépare la protéine de fusion.

6. Procédé selon la revendication 5, caractérisé en ce que, dans la structure génique, la séquence d'ADN I (voir appendice) code pour D' et/ou la séquence d'ADN (brin codant)

5'      AAA      GCA      AAG      GGC      3'

code pour X dans la structure génique, et/ou la structure génique est choisie de manière que la protéine de fusion soit insoluble et/ou la structure génique est contenue en phase dans un vecteur qui contient le promoteur, l'opérateur et le site de fixation des ribosomes du peptide L de l'opéron trp de *E. coli*.

7. Procédé selon la revendication 6, caractérisé en ce que le vecteur est un dérivé de pBR322, dans lequel le fragment allant du site Hind III en position 29 au site PvuII en position 2066 est éliminé hors de l'ADN de pBR322.

8. Structure génique codant pour des protéines de fusion selon l'une des revendications 1 à 4.

9. Vecteur, de préférence dérivé du plasmide pBR322, dans lequel le fragment allant du site Hind III en position 29 au site PvuII en position 2066 est éliminé hors de l'ADN de pBR322, contenant une structure génique selon la revendication 8.

10. Système d'expression, de préférence système de *E. coli*, contenant un vecteur selon la revendication 9.

11. Procédé pour la production d'une protéine d'eucaryote, caractérisé en ce que l'on sépare chimiquement ou enzymatiquement la séquence d'aminoacides Z d'avec une protéine de fusion selon l'une des revendications 1 à 4.

12. Plasmide pH 154/25 (fig. 3), pH 254 (fig.4), 30 pH 255 (fig. 4), pH 256 (fig. 5), pH 257 (fig. 5), pH 120/14, pK 150 (fig. 7), pK 160 (fig. 8), pK 170 (fig. 9), pK 180 (fig. 10), pH 154/25* (fig. 11), pH 256*, pH 120/14*, pK 150*, pK 170* et pInt 13 (fig. 12).


**Revendications** Pour l'Etat Contractant: AT

1. Procédé pour la préparation d'une protéine de fusion de formule générale (1)

$Met - X - D' - Y - Z$                  (1)

dans laquelle

# EP 0 211 299 B1

n est zéro ou 1,

X est une séquence de 1 à 12 aminoacides génétiquement codables,

D' est une séquence d'environ 70 aminoacides dans la région de la séquence d'aminoacides 23 - 93 du peptide D dans l'opéron trp de *E. coli*,

Y représente une séquence constituée d'un ou plusieurs aminoacides génétiquement codables, qui permet une séparation de la séquence d'aminoacides Z suivante, et

Z est une séquence d'aminoacides génétiquement codables, caractérisé en ce que l'on exprime dans une cellule hôte une structure génique codant pour cette protéine de fusion, puis on sépare la protéine de fusion.

2. Procédé selon la revendication 1, caractérisé en ce que l'on utilise une structure génique codant pour une protéine de fusion de formule 1, dans laquelle n est 1 et X comprend jusqu'à 5 aminoacides.

3. Procédé selon la revendication 1 ou 2, caractérisé en ce que l'on utilise une structure génique codant pour une protéine de fusion de formule 1, dans laquelle n est 1 et Lys-Ala se trouve à l'extrémité N-terminale dans X.

4. Procédé selon une ou plusieurs des revendications précédentes, caractérisé en ce que l'on utilise une structure génique codant pour une protéine de fusion de formule 1, dans laquelle Y contient à l'extrémité C-terminale Met, Cys, Trp, Arg ou Lys, ou l'un des groupes $(Asp)_m$-Pro ou Glu-$(Asp)_m$-Pro ou Ile-Glu-Gly-Arg, dans lesquels m représente 1, 2 ou 3, ou consiste en ces aminoacides.

5. Procédé selon une ou plusieurs des revendications précédentes, caractérisé en ce que l'on utilise une structure génique codant pour une protéine de fusion de formule 1 dans laquelle Z représente la séquence d'aminoacides de la proinsuline humaine ou d'une hirudine.

6. Procédé selon une ou plusieurs des revendications précédentes, caractérisé en ce que l'on utilise une structure génique codant pour une protéine de fusion de formule 1, dans laquelle la séquence d'ADN I (voir appendice) code pour D'.

7. Procédé selon la revendication 6, caractérisé en ce que dans la structure génique, la séquence d'ADN (brin codant)

5'      AAA     GCA     AAG     GGC     3'

code pour X.

8. Procédé selon une ou plusieurs des revendications précédentes, caractérisé en ce que la structure génique est choisie de manière que la protéine de fusion soit insoluble.

9. Procédé selon une ou plusieurs des revendications précédentes, caractérisé en ce que la structure génique est contenue en phase dans un vecteur qui contient le promoteur, l'opérateur et le site de liaison des ribosomes du peptide L de l'opéron-trp de *E. coli*.

10. Procédé selon la revendication 9, caractérisé en ce que le vecteur est un dérivé de pBR322, le fragment allant du site Hind III en position 29 au site PvuII en position 2066 étant éliminé hors de l'ADN de pBR322.

11. Procédé selon une ou plusieurs des revendications précédentes, caractérisé en ce que la cellule hôte est *E. coli*.

12. Procédé pour la production d'une protéine d'eucaryote, caractérisé en ce que l'on sépare chimiquement ou enzymatiquement la séquence d'aminoacides Z d'avec une protéine de fusion obtenue selon l'une des revendications 1 à 5.

**Claims** for the Contracting States: BE, CH, DE, FR, GB, IT, LI, LU, NL, SE

1. A fusion protein of the general formula

Met - $X_n$ - D' - Y - Z

in which

n is zero or 1,

X is a sequence of 1 to 12 genetically codable amino acids,

D' is a sequence of about 70 amino acids in the region of the sequence of amino acids 23 - 93 of the D-peptide in the trp operon of E. coli,

Y denotes a sequence of one or more genetically codable amino acids which permits the following amino acid sequence Z to be cleaved off, and

Z is a sequence of genetically codable amino acids.

13

2. A fusion protein according to claim 1, characterized in that n is one and X comprises up to 5 amino acids, Lys-Ala being preferably located at the N-terminal end of X.

3. A fusion protein according to claim 1 or 2, characterized in that Y contains at the C-terminal end Met, Cys, Trp, Arg or Lys or one of the groups $(Asp)_m$-Pro or Glu-$(Asp)_m$-Pro or Ile-Glu-Gly-Arg, in which m denotes 1, 2 or 3, or is composed of these amino acids or of one of these groups.

4. A fusion protein according to one or more of the preceding claims, characterized in that Z denotes the amino acid sequence of human proinsulin or of a hirudin.

5. A process for the preparation of the fusion proteins according to one or more of claims 1 to 4, characterized in that a gene structure coding for these fusion proteins is expressed in a host cell, preferably in a bacterium, particularly E. coli, and the fusion protein is separated off.

6. The process according to claim 5, characterized in that the DNA sequence I (appendix) codes in the gene structure for D', and/or the DNA sequence (coding strand)

$$5' \quad AAA \quad GCA \quad AAG \quad GGC \quad 3'$$

codes in the gene structure for X, and/or the gene structure is selected so that the fusion protein is insoluble, and/or the gene structure is contained in phase in a vector which contains the promoter, the operator and the ribosome binding site of the L-peptide from the trp operon of E. coli.

7. The process according to claim 6, characterized in that the vector is a derivative of pBR322, the segment from the Hind III site at position 29 to the PvuII site at position 2066 having been deleted from the pBR322 DNA.

8. A gene structure coding for fusion proteins according to claims 1 to 4.

9. A vector, preferably a derivative of the plasmid pBR322, the segment from the Hind III site at position 29 to the PvuII site at position 2066 having been deleted from the pBR322 DNA, containing a gene structure according to claim 8.

10. An expression system, preferably an E. coli system, containing a vector according to claim 9.

11. A process for the preparation of a eukaryotic protein, characterized by cleaving off, chemically or enzymatically, of the amino acid sequence Z from a fusion protein according to claims 1 to 4.

12. Plasmids pH 154/25 (Fig. 3), pH 254 (Fig. 4), pH 255 (Fig. 4), pH 256 (Fig. 5), pH 257 (Fig. 5), pH 120/14, pK 150 (Fig. 7), pK 160 (Fig. 8), pK 170 (Fig. 9), pK 180 (Fig. 10), pH 154/25* (Fig. 11), pH 256*, pH 120/14*, pK 150*, pK 170* and pInt 13 (Fig. 12).

**Claims** for the Contracting State: AT

1. A process for the preparation of a fusion protein of the general formula (1)

$$(1)$$

Met - $X_n$ - D' - Y - Z

in which

n   is zero or 1,
X   is a sequence of 1 to 12 genetically codable amino acids,
D'  is a sequence of about 70 amino acids in the region of the sequence of amino acids 23 - 93 of the D-peptide in the trp operon of E. coli,
Y   denotes a sequence of one or more genetically codable amino acids which permits the following amino acid sequence Z to be cleaved off, and
Z   is a sequence of genetically codable amino acids, characterized by expressing a gene structure which codes for these fusion proteins in a host cell, and separating the fusion protein.

2. The process according to claim 1, characterized in that use is made of a gene structure for a fusion protein of the formula 1 in which n is one and X comprises up to 5 amino acids.

3. The process according to claim 1 or 2, characterized in that use is made of a gene structure for a fusion protein of the formula 1 in which n is one and Lys-Ala is located at the N-terminal end of X.

4. The process according to one or more of the preceding claims, characterized in that use is made of a gene structure for a fusion protein of the formula 1 in which Y contains at the C-terminal end Met, Cys, Trp, Arg or Lys or one of the groups (Asp)m-Pro or Glu-(Asp)$_m$-Pro or Ile-Glu-Gly-Arg, in which m denotes 1, 2 or 3, or is composed of these amino acids.

5. The process according to one or more of the preceding claims, characterized in that use is made of a gene structure for a fusion protein of the formula 1 in which Z denotes the amino acid sequence of human proinsulin or of a hirudin.

6. The process according to one or more of the preceding claims, characterized in that use is made of a gene structure for a fusion protein of the formula 1 in which the DNA sequence I (appendix) codes for D'.

7. The process according to claim 6, characterized in that the DNA sequence (coding strand)

5'      AAA      GCA      AAG      GGC      3'

codes in the gene structure for X.

8. The process according to one or more of the preceding claims, characterized in that the gene structure is selected so that the fusion protein is insoluble.

9. The process according to one or more of the preceding claims, characterized in that the gene structure is contained in phase in a vector which contains the promoter, the operator and the ribosome binding site of the L-peptide from the trp operon of E. coli.

10. The process according to claim 9, characterized in that the vector is a derivative of pBR322, the segment from the Hind III site at position 29 to the PvuII site at position 2066 having been deleted from the pBR322 DNA.

11. The process according to one or more of the preceding claims, characterized in that the host cell is E. coli.

12. A process for the preparation of a eukaryotic protein, characterized by cleaving off, chemically or enzymatically, of the amino acid sequence Z from a fusion protein obtained according to claims 1 to 5.

# FIG.1

Met Lys Ala Lys

```
CGACA ATGAAAGCAAAGG : CCTTTGCTTTCATTGT
TGT TACTTTCGT TTCC : GGAAACGAAAGTAACAGC
           L'          StuI        (3)
```

FIG. 2

# FIG. 3

# FIG.4

pH 154/25   $\xrightarrow{\dfrac{\text{Bam HI}}{\text{Xma III}}}$   $\begin{array}{c} \_\_ G \\ \text{CCTAG} \\ \text{Bam HI} \end{array}$   $\begin{array}{c} \text{GGCCG} \_\_\_\_ \\ \text{C} \\ \text{Xma III} \end{array}$   (13)

(12)

Klenow, Ligase

Xma III (neu)

$\begin{array}{c} \_\_ \text{GGAT C} \vdots \text{GGCCG} \_\_\_ \\ \text{CCTAG} \vdots \text{CCGGC} \end{array}$

pH 254 (Tc$^S$)   (14)

pH 254   $\xrightarrow{\dfrac{\text{Mlu I}}{\text{Sal I}}}$   $\begin{array}{c} \_\_ G \\ \text{CAGCT} \\ \text{Sal I} \end{array}$   $\begin{array}{c} \text{CGCGT} \_\_\_ \\ \text{A} \\ \text{Mlu I} \end{array}$   (15)

(14)

Klenow, Ligase

Sal I (neu)

$\begin{array}{c} \_\_ \text{GTCGA} \vdots \text{CGCGT} \_\_\_\_ \\ \text{CAGCT} \vdots \text{GCGCA} \end{array}$

Mlu I (neu)

pH 255   (16)

# FIG.5

pH 154/25   $\xrightarrow{\dfrac{\text{Mlu I}}{\text{Eco RI}}}$   $\begin{array}{c} \_\_ G \\ \text{CTTAA} \\ \text{Eco RI} \end{array}$   $\begin{array}{c} \text{CGCGT} \_\_\_\_ \\ \text{A} \\ \text{Mlu I} \end{array}$ (17)

(12)

Eco RI (neu)

Klenow, Ligase   $\xrightarrow{\hspace{2cm}}$   $\begin{array}{c} \_\_ \text{GAATT} \vdots \text{CGCGT} \_\_\_\_\_ \\ \text{CTTAA} \vdots \text{GCGCA} \end{array}$   pH 256 (18)

pH 256 $\xrightarrow{\dfrac{\text{BamHI, Klenow}}{\text{Nru I, Ligase}}}$ $\begin{array}{c} \text{Bam HI (neu)} \\ \_\_ \text{GGATC} \vdots \text{CGA} \_\_\_\_ \\ \text{CCTAG} \vdots \text{GCT} \end{array}$   pH 257 (19)

(18)

# FIG. 6

pH 106/4 $\xrightarrow{\dfrac{Mst\ I}{Sal\ I}}$ ~ 495 bp - Fragment (22)
(11)

```
        Met  Lys  Ala  Lys
5'AC  GAA  TTC  ATG  AAA  GCA  AAG  G  3'
3'TG  CT T  AAG  TAC  T T T  CGT  T TC  C  5'
              (23)
```

(22)+(23) $\xrightarrow{\text{Ligase}}$ (24) $\xrightarrow{\dfrac{Eco\ RI}{Sal\ I}}$ (25)

(25)+(21) $\xrightarrow{\text{Ligase}}$

Ausschnitt aus pJ 120

```
       SD              EcoRI   Start Lys  Ala  Lys
5'··· CAC AGG AAA CAGAAT TC ATG AAA GCA AAG ··· 3'
3'··· GTG TCC T T TGTCT TAAG TAC T T T CGT T TC ··· 5'
```

# FIG. 7

# FIG. 8

pUC 13 $\xrightarrow{\text{1.Xma 1, 2.Klenow-P.}}$ großes Fragment (35)
(34) $\quad\quad$ 3. Sac 1

pK 150 $\xrightarrow{\dfrac{\text{Hind III}}{\text{Sac 1}}}$ Hind III – Hirudin – Sac 1
(33) $\quad\quad\quad\quad\quad\quad$ (36)

pH 106/4 $\xrightarrow{\dfrac{\text{Mst 1}}{\text{Hind III}}}$ Mst 1 – trp D' – Hind III
(11) $\quad\quad\quad\quad\quad\quad$ (37)

(35) + (36) + (37) $\xrightarrow{\text{Ligase}}$

(38)

FIG.9

EP 0 211 299 B1

FIG.10

pJF 118 (20) → 1.Eco RI 2.Klenow-P. 3.Sal I → Restplasmid (43)

pK 170 (42) → 1.Nco I 2.Klenow-P. 3.Hind III/Bam HI → NcoI⁻– trpD'– Hind III (44)
+
Hind III – Hirudin – Bam HI (45)

BamHI / Sal I → kleines Fragment (47)

(43) + (44) + (45) + (47) → Ligase

pUC 8 (46)

pK 180 (48)

17

FIG.11

# FIG.12

(51) $\dfrac{\text{Hind III}}{\text{Sal I}}$ ⟶ (10) + Restplasmid (52)

```
      (Ala) Trp  Glu  Asp  Pro  Met  Ile  Glu (Gly)(Arg)
   A   GCT  TGG  GAG  GAT  CCT  ATG  ATC .GAG  GG
            ACC  CTC  CTA  GGA  TAC  TAG  CTC  CCA  GCT
 (HindIII)           BamHI                          (Sal I)
                            (53)
```

(52) + (53) $\xrightarrow{\text{Ligase}}$

(54)